Europäisches Patentamt

European Patent Office

Office européen des brevets

(11) Publication number: **0 054 919**
A2

# EUROPEAN PATENT APPLICATION

(21) Application number: 81110538.6

(22) Date of filing: 17.12.81

(51) Int. Cl.³: **A 61 L 17/00**
// A61K35/14, A61K31/725

(30) Priority: 20.12.80 JP 180987/80

(43) Date of publication of application: 30.06.82
Bulletin 82/26

(84) Designated Contracting States: DE FR GB SE

(71) Applicant: NITTO ELECTRIC INDUSTRIAL CO., LTD.,
No 1-1-2, Shimohozumi Ibaraki-shi, Osaka-fu (JP)

(72) Inventor: Miura, Yoshiharu, 1-700 Megamiyama-cho
Koyoen, Nishinomiya Hyoto (JP)
Inventor: Aoyagi, Sadayoshi, 22-1-103,
Haramachi 1-chome, Suita Osaka (JP)
Inventor: Miyamoto, Kazuhisa, 33-13,
Higashiawaji 4-chome, Higashiyodogawa-ku Osaka (JP)

(74) Representative: von Kreisler, Alek, Dipl.-Chem. et al,
Deichmannhaus am Hauptbahnhof, D-5000 Köln 1 (DE)

(54) Artificial medical material.

(57) An artificial medical material with an anticoagulative
activity which comprises a carrier material, and at least
one of inhibitors of the coagulative system and at least
one of activators of the fibrinolytic system co-immo-
bilized thereon by a per se conventional procedure.

EP 0 054 919 A2

ACTORUM AG

0054919

# ARTIFICIAL MEDICAL MATERIAL

The present invention relates to an artificial medical material having an anticoagulative activity. More particularly, it relates to an artificial medical material useful for the prevention of blood coagulation, which comprises a carrier material and at least one of inhibitors of the coagulative system and at least one of activators of the fibrinolytic system co-immobilized thereon.

In surgical operations, a large amount of heparin is often administered to prevent the coagulation of blood. However, the administration of heparin in a large amount may sometimes cause lethal side effects such as mesentery bleeding. For overcoming such drawback, there has been recently proposed a method wherein heparin is immobilized on an appropriate carrier material such as a water-insoluble high polymer and blood is contacted therewith so that heparin may catch thrombin without entering into blood.

On the other hand, it has also been proposed an artificial medical material wherein either an inhibitor of the coagulative system or an activator of the fibrinolytic system is immobilized on a suitable carrier material. However, most of the carrier materials used for preparing such medical material do not have enough functional groups capable of combining with a large amount of the inhibitor or the activator which is required to show an satisfactory anticoagulative activity. Therefore, no artificial medical

- 2 -

0054919

material having a satisfactory anticoagulative property has been developed yet.

As a result of the extensive study on the prevention of blood coagulation and of formation of thrombus, it was found that an artificial medical material obtained by immobilizing both heparin and antithrombin III on a silicone resin or any other material coated with silicone exhibited a satisfactory anticoagulative activity and is therefore practically usable (cf. Japanese Patent Publication (unexamined) No. 38178/80). This is based on the findings that the co-immobilization of heparin and antithrombin III on a carrier material affords a superior anticoagulative activity to the immobilization of either of them, and that since a sufficient anticoagulative activity can be achieved even with small amounts of the anticoagulative substances immobilized on the carrier material, a remarkable anticoagulative activity can be imparted even to a carrier material having few functional groups.

The further extensive study based on the above findings has now revealed that co-immobilization of at least one of inhibitors of the coagulative system and at least one of activators of the fibrinolytic system on a carrier material affords a superior anticoagulative activity to the immobilization of either of them.

Thus, the present invention is to provide an artificial medical material having an anticoagulative activity, which comprises a carrier material, and at least one of inhibitors of the coagulative system and at least one of activators of the fibrinolytic system co-immobilized thereon.

As is well known, an inhibitor of the coagulative system means a substance capable of blocking any step of coagulative system and thus inhibiting blood coagulation. The representative inhibitors are heparin and antithrombin III. On the other hand, an activator of the fibrinolytic system represents a substance capable of activating a fibrinolytic system and thus accelerating the dissolution of thrombus formed by the activation of the coagulative system. The representative activator is urokinase. Therefore, the most preferred embodiment of the present invention is an artificial medical material which comprises a carrier material, and heparin and/or antithrombin III and urokinase co-immobilized thereon.

For the purpose of simplicity, the inhibitor of the coagulative system and the activator of the fibrinolytic system are hereinafter referred to as "coagulation inhibitor" and "fibrinolysis activator", respectively.

As understood from the above explanation, the artificial medical material of the invention has both an anticoagulative activity and a thrombus dissolving activity.

The artificial medical material of the invention can be prepared by immobilizing one or more of the coagulation inhibitors and one or more of the fibrinolysis activators on a carrier material according to a per se conventional procedure. It is not essential in the production of the artificial medical material that both agents are immobilized on a single carrier material. In other words, the medical material of the invention can also be the combination of a carrier material on which the coagulation inhibitor is immobilized and a carrier material on which the fibrinolysis activator is immobilized.

The carrier material may be selected from various conventional carrier materials useful for the immobilization of enzymes, so far as it is physiologically acceptable. Typical examples of such carrier material are silicone, polyvinyl alcohol, polyhydroxyethyl methacrylate, agarose, etc. Depending on the application or utilization of the artificial medical material, an appropriate carrier material having suitable physical properties may be used.

For preparation of the artificial medical material, any suitable procedure may be selected from various conventional methods for the immobilization of enzymes such as the carrier linkage method, the inclusion method and the suspension method. These and other methods are described in detail, for instance, in "Seikagaku Jikken Koza (Series of Experiments in Biochemistry)", Vol. 5, Method for Investigation of Enzymes, Part VI, Immobilized Enzymes, 1975.

The proportion of the amounts of the coagulation inhibitor and the fibrinolysis activator to be immobilized on the carrier material is not particularly limited, but it is usually in the range of 1 : 10 to 10 : 1 (weight ratio). Thus, in the case of the most preferred embodiment of the invention, the proportion of the amounts of antithrombin III, heparin and urokinase is 1 - 10 : 1 - 10 : 10 - 1 (weight ratio).

As previously stated, the artificial medical material on which the fibrinolysis activator is immobilized, as well as that bearing the coagulation inhibitor, is already known. Thus, the characteristic feature of the invention resides in the combined use of these different agents immobilized on one or more carrier materials.

It is surprising that the artificial medical material of the invention has a superior anticoagulative activity to the medical material on which either of the coagulation inhibitor or the fibrinolysis activator is immobilized. In other words, it is of unexpected nature that the artificial medical material of the invention has a synergistic or potentiation effect. The reason why such synergism or potentiation is produced is still not clear but may be assumed as follows:

The fibrinolysis activator, typically urokinase, does not directly dissolve a thrombus but serves, by reacting with plasminogen occurring in blood, to form plasmin which is a protease capable of dissolving the thrombus. Thus, the fibrinolysis activator acts indirectly and retardingly. To the contrary, the coagulation inhibitor such as heparin or antithrombin III combines directly with thrombin, which is an activated coagulative factor in blood. Thus, the coagulation inhibitor has a direct and immediate effect. On the other hand, the coagulation inhibitor combines with the coagulative factor and, therefore, its activity becomes weak gradually, whereas the fibrinolysis activator acts as a catalyst for activating plasminogen and, therefore, retains constant activity. Accordingly, these agents, when combined together, cooperate to compensate for their respective drawbacks, thereby giving the synergistic effect.

The artificial medical material of the invention may be prepared into any suitable form such as plate, powder, tube, fiber or mesh depending on its use. Further, the surface of the material can be smooth or coarse, as well as porous. When blood is contacted with the material which may be, for instance, in the shape of a blood flow tube or a reactor, thrombin and other activated factors in the coagulative system are readily caught thereby, and if thrombus is once formed, it is readily dissolved, thereby avoiding any possible blood coagulation.

The present invention will be hereinafter explained in detail by the following examples. The examples are only for the purpose of illustration and should not be construed as limiting the invention in any respect.

Example 1

Antithrombin III and heparin were immobilized on agarose, which had been previously activated by CNBr according to the method as disclosed in Y. Miura et al.: "Artificial Organs", 7, 193 (1978)).

One gram (dry weight) of agarose ("Sepharose 4B" manufactured by Pharmacia Fine Chemical Co., Ltd., Sweden) was repeatedly swelled and washed on a glass filter using 1 mM hydrochloric acid aqueous solution (300 ml) for 30 minutes. The swelled agarose was washed with deionized water (10 ml) to remove the hydrochloric acid and, then washed with a 0.1 M carbonate buffer (10 ml) having a pH of 8.0 and containing 0.5 M NaCl. Immediately after the washing, the swelled agarose was added to a solution of antithrombin III (0.5 mg) and heparin (0.5 mg) dissolved in a carbonate buffer (10 ml), and the resulting suspension was kept overnight at 4°C while stirring.

The agarose thus obtained, on which heparin and antithrombin III were immobilized, was washed on a glass filter successively with deionized water (20 ml), a 2.0 M NaCl aqueous solution (120 ml) and a 0.15 M NaCl aqueous solution, and preserved while being suspended in a 0.145 M NaCl aqueous solution.

In the same manner as above, each of urokinase (5 mg) and bovine serum albumin (10 mg) was respectively immobilized on an activated agarose. The resultant agarose bearing albumin was used as a control in the test described below.

- 8 -

0054919

The anticoagulative activity of each of the immobilized materials obtained above was determined as described below, according to the Chandler's rotating tube method (cf. A.B. Chandler: "Lab. Invest. 7, 110 (1958)).

The immobilized material (10 mg or 1 mg) and whole blood from an adult dog (800 µl, not treated with an anticoagulant) were placed into a ring-shaped tube (6 mmØ x 210 mm, made of vinyl chloride) connected with a silicone tube, and the time required for the coagulation of blood to complete is determined at room temperature. The results are shown in Table 1.

Table 1

| Immobilized material (Amount of immobilized agent/ amount of agarose) | Coagulation time time (min.) |
|---|---|
| Bovine serum albumin (100 µg/10 mg) (control) | 3.0 |
| Urokinase (50 µg/10 mg) | 3.5 |
| Antithrombin III + heparin (0.5 µg + 0.5 µg/1 mg) | 8.0 |
| Antithrombin III + heparin (0.5 µg + 0.5 µg/1 mg) + urokinase (50 µg/10 mg) | >30 |

0054919

From Table 1, it is understood that co-existence of antithrombin III and heparin retards the blood coagulation, but cannot completely inhibit the coagulation with the dose indicated, while addition of a small amount of urokinase-immobilized material to the above system remarkably prolongs the coagulation time (no substantial coagulation occurs).

Example 2

The same procedure as in Example 1 was carried out but using the different amounts of the immobilized materials. Further, an immobilized material wherein antithrombin III, heparin and urokinase (1 : 1 : 10) were immobilized on a single carrier material (5 mg) was also tested in this example. The results are shown in Table 2.

- 10 -

0054919

Table 2

| Immobilized material (Amount of immobilized agent/ amount of agarose) | Coagulation time time (min.) |
|---|---|
| Physiological salt solution (control) | 3.5 |
| Bovine serum albumin (200 μg/20 mg) (control) | 3.5 |
| Urokinase (100 μg/20 mg) | 4.5 |
| Antithrombin + heparin (2.5 μg + 2.5 μg/5 mg) | 10.0 |
| Antithrombin III + heparin (2.5 μg + 2.5 μg/5 mg) + urokinase (100 μg/20 mg) | > 30 |
| Antithrombin III + heparin + urokinase (2.5 μg + 2.5 μg + 25 μg/5 mg) | > 30 |

From Table 2, it is understood that immobilized material wherein the antithrombin III, heparin and urokinase were all immobilized on the single carrier completely inhibits blood coagulation, even though the amount of urokinase is extremely decreased as compared with the amount of urokinase in the combination of the material bearing the antithrombin III and heparin and the material bearing the urokinase.

- 11 -

00549l9

What is claimed is:

1. An artificial medical material having an anticoagulative activity, which comprises a carrier material, and at least one of inhibitors of the coagulative system and at least one of activators of the fibrinolytic system co-immobilized thereon.

2. The artificial medical material acccording to claim 1, wherein the inhibitors are heparin and antithrombin III and the activator is urokinase.

3. The artificial medical material according to claim 2, wherein the proportion of the amounts of antithrombin III, heparin and urokinase is in the range of 1 - 10 : 1 - 10 : 10 - 1.